# EUROPEAN PATENT SPECIFICATION

(11) **EP 2 000 033 B1**
(45) Date of publication and mention of the grant of the patent: **12.01.2011**
(21) Application number: 08425189.1
(22) Date of filing: 21.03.2008
(51) Int. Cl.: A21D 8/04, C12R 1/865

(54) **Microbial starter comprising Saccharomyces cerevisiae strains for baking**
Mikrobieller Starter, welcher Saccharomyces cerevisiae Stämme enthält, für die Herstellung von Backwaren
Starter microbien, comprenant des souches de Saccharomyces cerevisiae, pour la panification

(30) Priority: 06.06.2007 IT RM20070317
(43) Date of publication of application: 10.12.2008
(73) Proprietor: Parco Scientifico e tecnologico della sicilia, Z.I. Blocco Palma I Stradale Agnelli Ang. Via Lancia 95030 Catania (IT)
(72) Inventor: Catara, Antonino, 95030 Catania (IT); Giannone, Virgilio, 95030 Catania (IT); Paparone, Nicoletta, 95030 Catania (IT); Longo, Chiara, 95030 Catania (IT)
(74) Representative: Gitto, Serena

(56) References cited:
- PYLER EJ: "Baking science and technology, pp. 203-212" 1988, SOSLAND PUBLISHING COMPANY , KANSAS CITY, USA , XP002541926 * the whole document *

## Description

The present invention concerns a microbial starter based on selected yeasts for the production of bakery products, preferably bread. Particularly, the invention concerns the use of "natural yeast" as yeasting agent for the bread production: The use of selected yeasts allows an improvement of yeasting power and organoleptic and nutritional characteristics of bakery products compared to those resulting from baker's yeast to be obtained.

The bread-making needs of yeast, whose function is the fermentation of dough resulting in volume increase and lighter and more easy digestible bread. In bread-making Saccharomyces cerevisiae cultures, which through the fermentation of glucose, derived from starch hydrolysis, produce ethanol and CO₂, are used.

There are essentially three types of yeasts:
- industrial compressed yeast;
- chemical yeast;
- natural or acid paste yeast.

Yeast, both "commercial" or "natural", consists of one or more species of microscopic fungi able to use wheat flour for living and reproducing, resulting in the production of various waste products in addition to gas. These waste compounds together with accompanying gas are responsible of the bread yeasting and resulting aroma.

### Industrial compressed yeast

Usually it is marketed as 25 g small bars to be stored in cold and dry place, or freeze-dried sachets corresponding to a 25 g fresh dose. It consists of Saccharomyces cerevisiae species yeasts artificially selected both for the taste and, above all, to provide a stable and reproducible activity.

Saccharomyces cerevisiae, belonging to Ascomycetes class, is very important due to the functions which it carries out, in that it is able to ferment monosaccharides and di-saccharides resulting in the production of ethanol and carbon dioxide. The production of this gas is exploited in many industrial processes like bread yeasting.

Before the commercial production of yeast, all the bread was produced from natural yeasts

### Chemical yeast

It is the yeast usually used for the production of pastries and salty cakes, but it can be used also for the bread production. It is mainly constituted of sodium or ammonium bicarbonate, alkaline substances (that is suitable for acid neutralization) associated with acid substances (usually potassium tartrate or tartaric acid), resulting in a neutral compound. These compounds co-react producing carbon dioxide, resulting in kneaded wheat flour yeasting.

### Natural or wild or acid yeast

Acid dough, used at handmade level for typical bread production, consists of a complex biological system, wherein yeasts (Saccharomyces cerevisiae, Candida krusei, Hansenula anomalous, etc.) are present, the activity of which affects the yeasting process, and omo- and hetero-yeasting lactic bacteria (Lactobacillus sanfranciscensis, Lactobacillus brevis, Lactobacillus fermentum, Lactobacillus pontis, Lactobacillus panis, Lactobacillus mindensis, Lactobacillus plantarum, etc.) which, together with the products of their metabolism, positively affect the structural and organoleptic characteristics of finished products, in addition to shelf-life thereof. Therefore various species and strains of yeasts, naturally occurring in the environment, are present. They are viable at temperature conditions more variable than commercial yeast, can survive in more acid environments and provide bread with typical peculiar aromas of each yeast.

Natural: because they can be obtained from natural yeast, simply from wheat flour and water, capturing yeasts occurring in the atmosphere. Therefore yeasts with various characteristics according to the atmosphere wherein they have been "captured" will be produced.

Wild: because they are not selected by humans, differently than shop marketed small bar yeasts, but atmosphere naturally occurring ones.

Acid: because these yeasts confer to the bread a more or less acid taste, according to yeast type. Based on the used technology production, acid dough are classified according to three different typologies:
I type: dough obtained using traditional production techniques and characterised by continuous propagations at room temperature (20-30°C) in order to have viable microorganisms. Lactobacillus sanfranciscensis and Lactobacillus pontis are predominant lactic bacteria, the presence of Lactobacillus fructivorans, Lactobacillus fermentum, Lactobacillus brevis and Lactobacillus farciminis has been also detected, which yeasts generally are used for the production of traditional products with three steps fermentation processes, French bread, san francisco bread and panetton represent examples of products obtained from this dough;
II type: dough produced using longer fermentation times (longer than 5 days) at temperature higher than 30°C, with the addition of commercial yeast, essential for the yeasting, they allow the fermentation process time to be reduced (one step process). This type of sourdough, mainly used for industrial processes, is characterised by the large presence of Lactobacillus panis and Lactobacillus pontis, and in a smaller extent of Lactobacillus reuteri, Lactobacillus johnsonii, Lactobacillus sanfranciscensis, Lactobacillus fermentum, Lactobacillus delbrueckii, Lactobacillus acidophilus, Lactobacillus brevis, Lactobacillus amylovorus and Lactobacillus frumenti;
III type: dough obtained with traditional fermentations, dehydrated and generally used to acidify or provide aroma to the product. Also for this dough type it is necessary, for the yeasting, the addition of commercial yeast.

In the light of above described, it is therefore apparent the need to provide new yeast strains for the preparation of bakery products suitable to reduce the yeasting times and thus increase the hourly production in comparison to commercially available yeasts.

The authors of the present invention have isolated and selected three yeast strains to be used alone or in combination displaying surprising functional characteristics in terms of yeasting power during the first 2 hours (greater volumetric increment to by 20 % compared to commercial yeast obtained dough) whose use as microbial starter in processes for bakery products production allows yeasting times to be remarkably reduced and correspondingly hourly production to be increased

Further advantages from the use of microbial starter, object of the present invention, are:
- use of smaller yeast doses in the production steps;
- use dose as low as 1 % (w/w) also at smaller concentration/g of dried substance;
- volumetric increase higher than obtained with commercially available starters;
- reduction of working times;
- potentially use of yeast strains also in liquid form without the re-hydration step.

It is therefore a specific object of the present invention a microbial starter for bakery products based on Saccharomyces cerevisiae yeast strains comprising or consisting of at least one of yeast strains selected from the group consisting of Saccharomyces cerevisiae DSM 18864, Saccharomyces cerevisiae DSM18865 and Saccharomyces cerevisiae DSM 18866 (all strains deposited on 14th December 2006 at DSMZ) or a combination thereof.

Preferably, said microbial starter comprises or consists of using individual yeast strain or combination of Saccharomyces cerevisiae DSM 18864 and Saccharomyces cerevisiae DSM 18865 or Saccharomyces cerevisiae DSM 18865 and Saccharomyces cerevisiae DSM 18866, respectively.

In a particularly preferred embodiment of the present invention in the microbial starter according to the invention the strains are at 1:1 ratio at a cell density of 1x10¹⁰ cfu/g at fermentation start.

According to alternative embodiments of the invention the strains can be freeze-dried, frozen, dried or stored in liquid form. In this latter case it is not necessary their re-hydration before the use.

It is a further object of the present invention the use of the microbial starter as before defined for wheat flour yeasting and production of bakery products, preferably bread. Preferably, the wheat flours are selected from the group consisting of hard wheat semola wheat flour, soft wheat flour 0 and 00 and mixtures thereof. According to a preferred embodiment of the present invention the starter use dose is equal to 1 % (w/w) also at concentrations lower than usual for commercially available starters (7x10¹⁰ cfu/g).

The present invention further refers to a process for the preparation of microbial starter for bakery products as above defined comprising the following steps of:
a) culture propagating of at least one of the yeast strains selected from the group consisting of Saccharomyces cerevisiae DSM 18864, Saccharomyces cerevisiae DSM 18865 and Saccharomyces cerevisiae DSM 18866 (all strains deposited on 14th Dicember 2006 at DSMZ) or combination thereof;
b) wheat flour kneading (preferably 100 % semola or 80 % semola and 20 % wheat flour or 50 % semola and 50 % wheat flour and water (50 - 60 weight %, preferably 50 weight % of total dough depending on the wheat flour humidity) containing the culture of a) step bacterial strains at cellular density of 1x10¹⁰ cfu/g at 1:1 strain ratio;
c) allowing the fermentation to be performed for 1.15-1.30 hours at 35°-40°C.

According to an alternative embodiment of the invention the process for the preparation of the microbial starter for bakery products further comprises a d) step for drying, freeze-drying, drying or emulsion in liquid phase of the starter obtained in a) step.

It is a further object of the present invention a microbial starter composition by using the process as above defined according to a) - c) or a) - d) steps thereof.

A further object of the present invention is a process for the preparation of bakery products, preferably bread, comprising the following steps of:
a) kneading wheat flour (semola 100 % or wheat flour 00 100 %, preferably semola 80 % and wheat flour 20 %) with 50 - 60 weight % water, preferably 50 %, containing microbial starter inoculum for bakery products according to the present invention or obtainable using the process for the preparation of microbial starter as above defined;
b) allowing the fermentation to be carried out for approximately 1.15-1.30 hours at 35°-40°C;
c) cooking for 40-50 minutes at 220-240°C.

The present invention now will be described by illustrative but not limitative way, according to preferred embodiments thereof, with particular reference to enclosed drawings, wherein:
figure 1 shows the yeasting power after 4 hours of yeast strains in hard wheat semola (Mongibello cultivar);
figure 2 shows the yeasting power after 12 hours of yeast strains in hard wheat semola (Mongibello cultivar);;
figure 3 shows the yeasting power after 4 hours of bread-making yeast strains in hard wheat semola (commercial cultivar);
figure 4 shows the yeasting power after 12 hours of bread-making yeast strains in hard wheat semola (commercial cultivar);
figure 5 shows the yeasting power comparison of yeast strains selected from criscenti (sourdough yeast) or manmade mothers;
figure 6 shows the yeasting power comparison of yeast strains selected according to the invention, i.e. S. cerevisiae DSM18864, DSM18865, DSM18866 and national (compressed baker's yeast) and international (powdered baker's yeast) commercial starters;

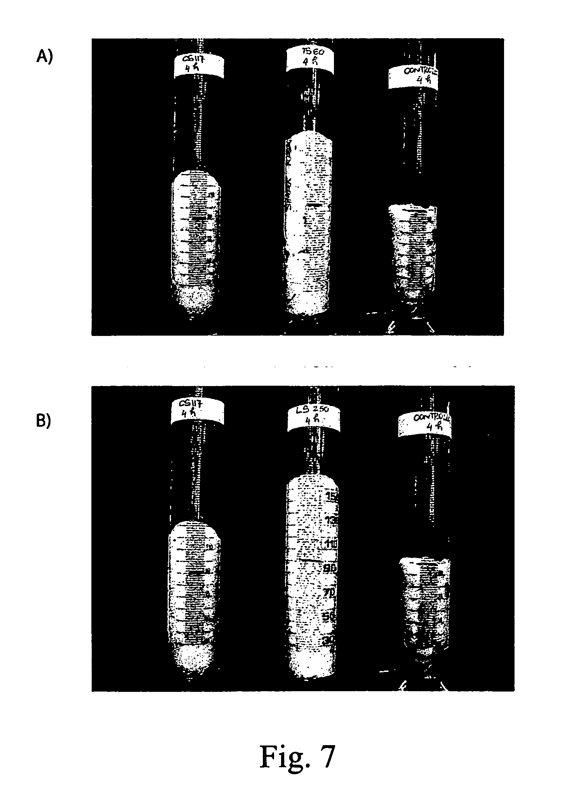

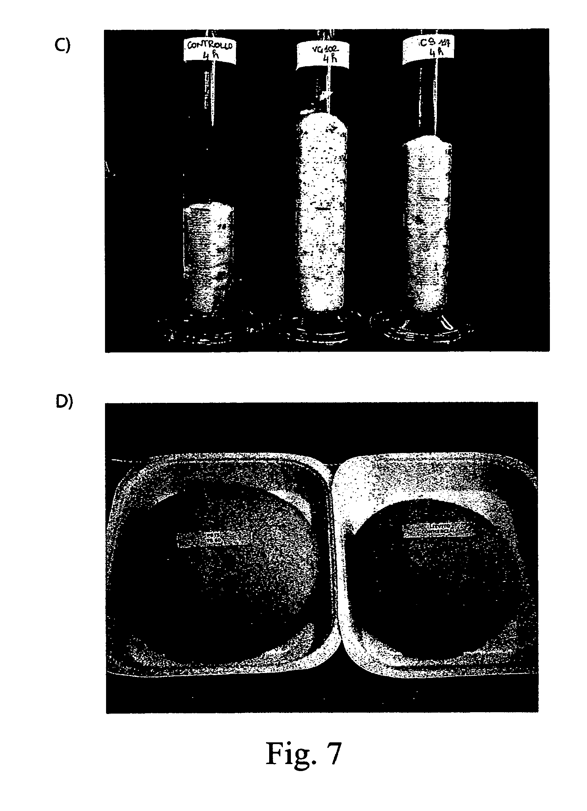

figure 7 shows the yeasting power comparison of bread-making yeast strains according to the invention and commercial ones; A panel shows commercial yeast, TS60, named DSM 18865, yeast strain and semola and water in the left, middle and right parts, respectively; B panel shows commercial yeast, LS250, named DSM 18864, yeast strain and semola and water in the left, middle and right parts, respectively; C panel shows commercial yeast, VG102, named DSM 18866, yeast strain and semola and water in the left, middle and right parts, respectively; panel D shows the power yeasting comparison in hard wheat semola dough between TS60, named DSM 18865, yeast strain (left) and commercial yeast (right).

### EXAMPLE 1: Isolation, characterisation and selection of S cerevisiae yeast strains according to the invention

### MATERIALS AND METHODS

### Sampling

During March 2003-June 2004 period samplings relating to home-made formulations of naturally occurring yeasts have been made directly in East Sicily areas with bread-making aptitude. 500 g "Criscenti" or sourdough samples have been brought at Laboratory, 100 g aliquots of the same have been sampled and stored at -18°C. Sourdough or natural yeasts are dough obtained from spontaneous fermentation by means of not selected microorganisms occurring in wheat flour and represent the natural microbial inoculum used for the production of characteristic bakery products.

Approximately 17, 23 and 11 samples in Ragusa, Syracuse and Catania provinces have been collected, respectively.

### Yeast Isolation and identification

"criscenti" sourdough manmade samples have been thawed in the previous evening, 25 g of sample have been homogenised in Stomacher with 225 ml of sterile physiological solution. From thus prepared sample (dilution 10⁻¹) serial dilutions until 10⁻⁷ (De Medici et al., 1996) in physiological solution have been prepared. 100 ml aliquots have been spread using a spatula on Petri discs containing chloramphenicol supplemented SDA (Oxoid) in duplicate and incubated for 4-5 days at 26-28°C; after 3-4 incubation days, for each seed 4 colonies have been sampled and transferred in culture tubes, cultured at 25°C and then stored at 4°C. A strain bank comprising about 100 strains has been constituted.

In order to identify S. cerevisiae sp. strains the following characteristics will be examined:
- *morphology of the colonies observed on plate medium;*
- *sporification capacity on agar-acetate medium:* cells grown in YPD medium are transferred into agar acetate sporification medium (1 % potassium acetate, 1.6 % agar) and incubated at 25°C; under these conditions the cells, in a few days, are subjected to meiosis resulting in the formation of four asco spores, observable by means of a microscope; the sporification is essential for the control of the strain stability and genetic characterisation and their improvement by cross-breeding schedules;
- *growth at 37°C:* cell suspensions are smeared on YPD medium and incubated at 37°C; after 3 days the growth will be observed.
- *sugar fermentation capacity (maltose, saccharose, raffinose and galactose);*
- *no-growth in agar-lisyne medium:* the test has been carried out in Lysina medium (Oxoid) supplemented with 50 % potassium lactate and 10 % lactic acid; this medium contains lysine amino acid as unique nitrogen source; when cells of a strain belonging to S cerevisiae species are placed in this medium, they do not grow because the strains of this species are unable to use lysine as unique nitrogen source.

Yeast isolates and their origin are illustrated to in the following Table 1:

**Table 1**

| Strains | Source | N° Isolates |
|---|---|---|
| VG | Giarre (CT) | 13 |
| TS | Ispica (RG) | 17 |
| LS | Ispica (RG) | 14 |
| GP | Pachino (SR) | 10 |
| CAB | Belpasso (CT) | 10 |
| LZ e DLS | Belpasso (CT) | 28 |

### Yeast strain storage

For strains storage, 1.5-1.8 ml aliquots of YPD agar medium into 2 ml cryotubes have been transferred. Previously isolated strains have been transferred into the tubes by means of sterile loop by superficially smearing and subsequently infission ("clarion spout"). At last the tubes have been cultured for 48 hours in thermostatic bath at 25°C , before the storage at 4°C. The storage period is approximately 1-2 years.

The storage at -20°C has been then carried out using glycerolate nutrient broth with 20 % glycerol.

### Physiological and biochemical characterisation

Physiological and biochemistry characterisation allow to identify yeast from a species-specific point of view and evaluate qualitatively and quantitatively advantageous features for the fermenting and bread-making process. Following features have been examined:

### - Production of H₂S

The production of hydrogen sulphide is estimated by staining degree of the cells when they are replicated and grown in BIGGY-Agar medium containing bismuth ion. The hydrogen sulphide production from the medium leads to the generation of bismuth sulphide resulting in brown stained cells. The staining degree depends on the amount of yeast produced hydrogen sulphide and is estimated as increasing values from 0 to 4, 0 = no staining, 4 = high staining.

### - Maltose fermentation

Test tubes containing 10 ml of YNB broth have been inoculated using 100 µl pre-culture from 24 hour over-night yeast culture carried out in YPD broth so as to obtain the same cell concentration (10⁶ ufc/ml) in all test-tubes. Each test-tube contains 6 % maltose as unique carbon source and the fermentation is estimated by CO₂ development (inverted Durham bellflower).

The tests-tube are incubated at 28°C for 48 hours.

After 48 hours it is possible to observe the fermentation occurrence with CO₂ development and consequent tested carbohydrate metabolization.

### - Lactic acid resistance

The test is carried out by adding to 100 ml YPD broth, 2.5 % of 80 % lactic acid after cold 0,2 µm filter sterilization thereof. The over-night culture in sterile eppendorf tubes is prepared and the growth was allowed at 28°C for 24 hours. After 24 hours eppendorf tubes are centrifuged so as the pellet is settled out and the supernatant is removed. After over-night culture replacement it is inoculated in sterile eppendorf tubes containing 2.5 ml YPD broth containing filtered lactic acid, a yeast loop of each collected strain and finally it is incubated at 28°C for 48 hours.

### Molecular characterisation of yeasts

The molecular characterisation has been carried out using the so-called A.R.D.R.A. (amplified ribosomal DNA restriction analysis) technique. It is a species-specific identification technique using ribosomal DNA, as important genetic marker, being ribosome in all the organisms. rDNA molecules show highly conserved stretches, with high homology degree among all the organisms, alternated to inner spacing regions showing elevated polymorphism degree among different species. The technique is based on the amplification of ITS regions by PCR, using primers specific for two highly conserved regions located at 3' and 5' ends of 18 S and 25 S genes, respectively.

The amplification of the whole region comprising two inner spacers ITS1 and ITS4 and 5.8S gene produces characteristic DNA fragments. The length of amplified DNA fragments is different for different yeast species. In fact the amplification product is approximately 850 bp for Saccharomyces cerevisiae, S. pastorianus and S.bayanus meanwhile for other Saccharomyces species it is variable from 660 to 760 bp.

The amplified DNA fragments then are cut using endonuclease and restriction profiles are compared.

### RESULTS

Results of the selection tests and biochemical characterisation of the bread-making yeast strains are reassumed in the following Table 2.

**Table 2**

| ***Code*** | ***Species*** | ***SPO*** | ***Maltose 6%*** | ***LYS*** | ***H₂S*** | ***Lactic acid 2%*** |
|---|---|---|---|---|---|---|
| ***DSM 18866*** | *C. kefyr* | + | + | - | 2 | + |
| ***VG 111*** | Elonged cells | - | - | + | 4 | + |
| ***VG 144*** | *S. cerevisiae* | + | - | - | 2 | + |
| ***VG 170*** | *S. cerevisiae* | + | - | - | 2 | + |
| ***VG191*** | *S. cerevisiae* | + | - | - | 2 | + |
| ***VG 246*** | *S. cerevisiae* | + | - | - | 2 | + |
| ***VG 257*** | *S. cerevisiae* | + | - | - | 2 | + |
| ***VG 260*** | *C. riferii* | + | - | + | 4 | - |
| ***VG 272*** | *S. cerevisiae* | + | - | - | 2 | + |
| ***VG 311*** | *S. cerevisiae* | + | - | - | 2 | + |
| ***VG 341*** | *S. cerevisiae* | + | - | - | 2 | + |
| ***VG 654*** | *S. cerevisiae* | + | + | - | 2 | + |
| ***VG 923*** | *S. cerevisiae* | + | - | - | 2 | + |
| ***TS 11*** | *S. cerevisiae* | + | + | - | 2 | + |
| ***TS 20*** | *S. cerevisiae* | + | - | - | 2 | + |
| ***TS44*** | *S. cerevisiae* | + | - | - | 2 | + |
| ***TS 50*** | *S. cerevisiae* | + | + | - | 2 | + |
| ***DSM 18865*** | *S. cerevisiae* | + | - | - | 2 | + |
| ***TS 81*** | *S. cerevisiae* | + | - | - | 2 | + |
| ***TS 94*** | *S. cerevisiae* | + | - | - | 2 | + |
| ***TS 101*** | *S. cerevisiae* | + | - | - | 2 | + |
| ***TS 111*** | *S. cerevisiae* | + | - | - | 2 | + |
| ***TS 122*** | *S. cerevisiae* | + | + | - | 2 | + |
| ***TS 152*** | *S. cerevisiae* | + | - | - | 2 | + |
| ***TS 162*** | *S. cerevisiae* | + | - | - | 2 | + |
| ***TS 192*** | *S. cerevisiae* | + | - | - | 2 | + |
| ***TS 222*** | *S. cerevisiae* | + | - | - | 2 | + |
| ***TS 267*** | *S. cerevisiae* | + | + | - | 2 | + |
| ***TS 289*** | *S. cerevisiae* | + | - | - | 2 | + |
| ***TS 290*** | *S. cerevisiae* | + | + | - | 2 | + |
| ***LS 33*** | *S*. *cerevisiae* | + | + | - | 2 | + |
| ***LS 41*** | *S. cerevisiae* | + | + | - | 2 | + |
| ***LS 70*** | *S. cerevisiae* | + | + | - | 2 | + |
| ***LS 92*** | *S. cerevisiae* | + | + | - | 2 | + |
| ***LS 129*** | *S. cerevisiae* | + | + | - | 2 | + |
| ***LS 131*** | *S. cerevisiae* | + | + | - | 2 | + |
| ***LS 150*** | *S*. *cerevisiae* | + | + | - | 2 | + |
| ***LS 184*** | *S*. *cerevisiae* | + | + | - | 2 | - |
| ***LS 190*** | *S*. cerevisiae | + | + | - | 2 | + |
| ***LS 202*** | *S. cerevisiae* | + | + | - | 2 | |
| ***LS 243*** | *S. cerevisiae* | + | + | - | 2 | + |
| ***DSM 18864*** | *S. cerevisiae* | + | - | - | 2 | + |
| ***LS 276*** | *S. cerevisiae* | + | - | - | 2 | + |
| ***LS 295*** | *S. cerevisiae* | + | - | - | 2 | + |
| ***GP 23*** | *S. cerevisiae* | + | + | - | 2 | + |
| ***GP 49*** | *S*. *cerevisiae* | + | + | - | 2 | + |
| ***GP 92*** | *S. cerevisiae* | + | + | - | 2 | + |
| ***GP 138*** | *S. cerevisiae* | + | + | - | 2 | + |
| ***GP 176*** | *S. cerevisiae* | + | + | - | 2 | + |
| ***GP 211*** | *S. cerevisiae* | + | + | - | 2 | + |
| ***GP 258*** | *S. cerevisiae* | + | + | - | 2 | + |
| ***CAB 01*** | *S. cerevisiae* | + | - | - | 2 | + |
| ***CAB 02*** | *S. cerevisiae* | + | - | - | 2 | + |
| ***CAB 03*** | *S*. *cerevisiae* | + | + | - | 2 | + |
| ***CAB 04*** | *S. cerevisiae* | + | - | - | 2 | + |
| ***CAB 05*** | *S. cerevisiae* | + | - | - | 2 | + |
| ***CAB 06*** | *S. cerevisiae* | + | - | - | 2 | + |
| ***CAB 07*** | *S. cerevisiae* | + | - | - | 2 | + |
| ***CAB 08*** | *S. cerevisiae* | + | - | - | 2 | + |
| **LZ 1+** | *S.cerevisiae* | + | + | - | 3 | + |
| **LZ 2+** | *S.cerevisiae* | + | + | - | 3 | + |
| **LZ 4+** | *S.cerevisiae* | + | + | - | 3 | + |
| **LZ 5+** | *S.cerevisiae* | + | + | - | 1 | + |
| **LZ 6+** | *S.cerevisiae* | + | + | - | 3 | + |
| **LZ 7** | *S.cerevisiae* | + | + | - | 3 | + |
| **LZ 9** | *S.cerevisiae* | + | + | - | 3 | + |
| **LZ 10** | *S.cerevisiae* | + | + | - | 2 | + |
| **LZ 11** | *S.cerevisiae* | + | + | - | 2 | + |
| **DLS 01** | *S.cerevisiae* | + | + | - | 2 | + |
| **DLS 02** | *S.cerevisiae* | - | + | - | 2 | + |
| **DLS 03** | *S. cerevisiae* | + | + | - | 2 | + |
| **DLS 04** | *S.cerevisiae* | - | + | - | 2 | + |
| **DLS 05** | *S.cerevisiae* | + | + | - | 2 | + |

From this first step 20 yeast strains, all belonging to S. cerevisiae species, representing various geographic areas and with interesting technological properties have been identified.

### Tests on semola yeasting power

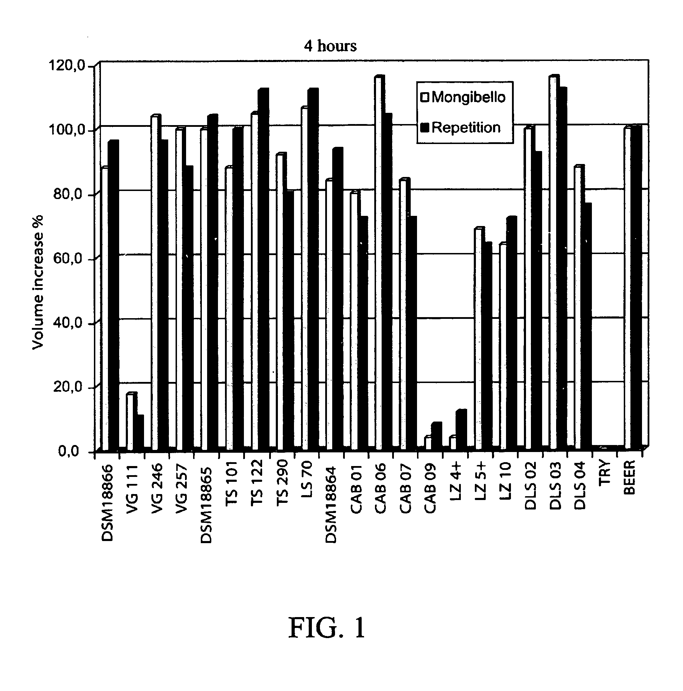

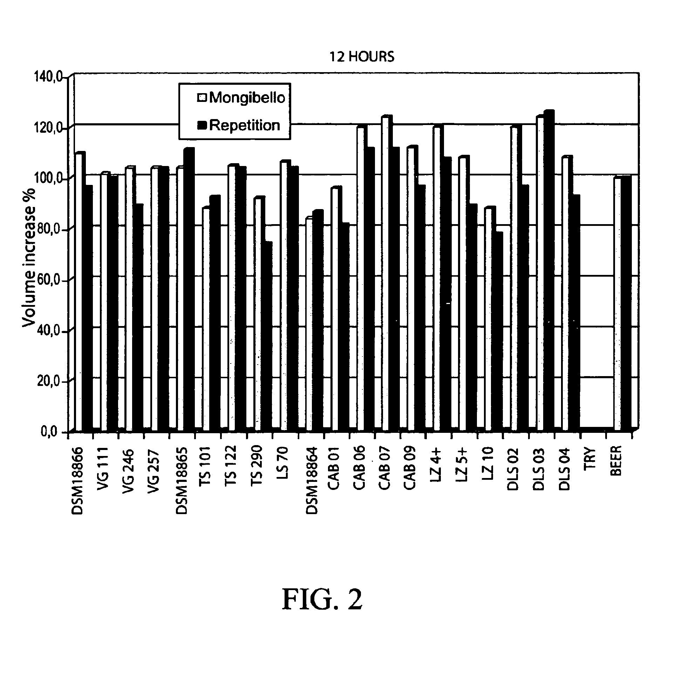

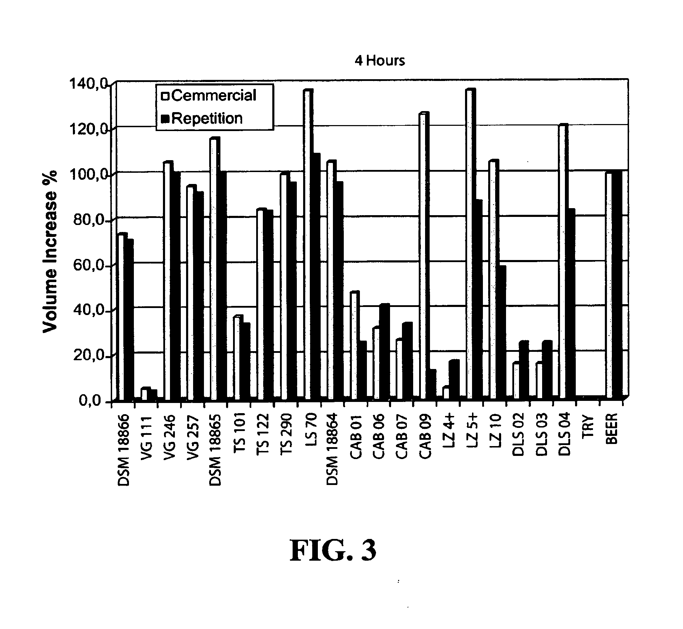

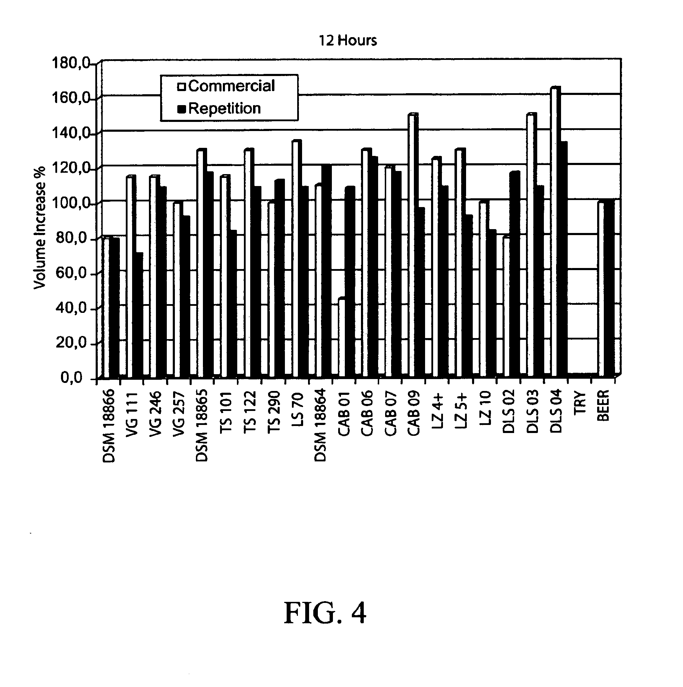

Yeasting tests have been carried out on dough obtained with Mongibello hard wheat semola (Figures 1-2) and commercial type with the addition of 50 % water and 0.85 % NaCl (Figures 3-4). Figures 1-4 show yeasting power of bread-making yeast strains after 4 and 12 hours respectively in Mongibello hard wheat semola or commercial type. For the preparation chopin alveographe kneader has been used. Kneading time was 5 minutes. Previously selected microorganisms have been added to dough after broth culture centrifugation, supernatant elimination and 0,5 g pellet sampling; added amounts have been calculated as cell wet weight/g of yeast. Each test has been carried out by preparation of 10 small bars containing selected strains, one containing baker's yeast and one containing only physiological solution with 0.85 % NaCl and semola (control).

At kneading end 100 g of the product have been compressed on the bottom of 250 ml graded cylinder, with surface sealing in order to avoid CO₂ escape, then incubated at 30°C. Every half hour the volume increase of the dough until the fourth and then twelfth hours has been monitored. All the tests have been performed in duplicate.

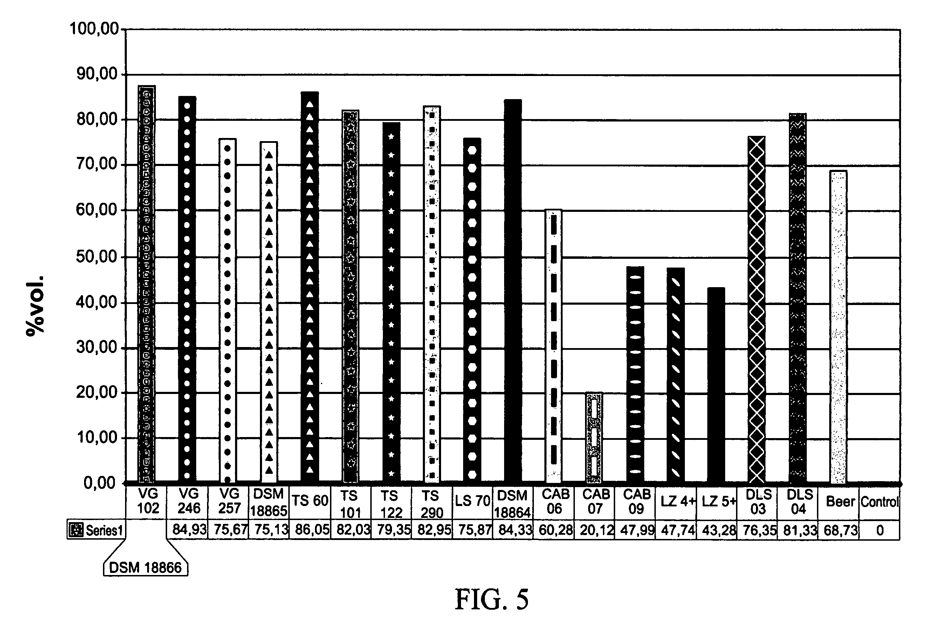

The tests allowed some yeast strains, interesting with respect to yeasting quickness and appreciable aromatic modification induced in the dough after 24 hours to be identified. Then the yeast strains have been compared among them employing commercial hard wheat semola and using in each test a bread-making yeast as comparison and a control (dough without yeast) as shown in the following Table 3 and Figure 5.

**Table 3**

| Code | Maltose | Replicate 1 | Replicate 2 | Replicate 3 | Mean | Volume increase % |
|---|---|---|---|---|---|---|
| DSM 18866 | + | 88.90 | 80.00 | 93.30 | 87.40 | 87.40 |
| VG 246 | + | 93.20 | 80.00 | 81.60 | 84.93 | 84.93 |
| VG 257 | + | 68.70 | 84.40 | 73.91 | 75.67 | 75.67 |
| TS 50 | + | 95.30 | 50.50 | 79.59 | 75.13 | 75.13 |
| DSM 18865 | - | 93.50 | | 78.60 | 86.05 | 86.05 |
| TS 101 | - | 89.05 | | 75.00 | 82.03 | 82.03 |
| TS 122 | - | 86.60 | | 72.10 | 79.35 | 79.35 |
| TS 290 | - | 82.60 | | 83.30 | 82.95 | 82.95 |
| LS 70 | - | 83.00 | 62.00 | 82.61 | 75.87 | 75.87 |
| DSM 18864 | - | 82.00 | 86.00 | 85.00 | 84.33 | 84.33 |
| CAB 06 | + | 51.00 | | 69.56 | 60.28 | 60.28 |
| CAB 07 | - | 17.00 | 7.00 | 36.36 | 20.12 | 20.12 |
| CAB 09 | + | 44.40 | 22.50 | 77.08 | 47.99 | 47.99 |
| LZ 4+ | - | 28.00 | 50.00 | 65.22 | 47.74 | 47.74 |
| LZ 5+ | + | 50.00 | 12.50 | 67.34 | 43.28 | 43.28 |
| DLS 03 | - | 87.20 | 74.50 | 67.35 | 76.35 | 76.35 |
| DLS 04 | - | 86.70 | 86.70 | 70.59 | 81.33 | 81.33 |
| Baker's | + | 72.00 | 66.00 | 68.20 | 68.73 | 68.73 |
| Control | | 0.00 | 0.00 | 0.00 | | 0 |

The evaluations allowed three strains (TS 60, LS 250 and VG 102) with yeasting power higher than commercial yeast to be identified. Said strains have been deposited at DSMZ, on 14 December 2006, with the following deposit numbers:
Saccharomyces cerevisiae LS 250 = DSM 18864
Saccharomyces cerevisiae TS 60 = DSM 18865
Saccharomyces cerevisiae VG 102 = DSM 18866.

### Comparison of yeasting power with commercial starters

Selected wine yeast strains have been compared, form point of view of their yeasting power, with two commercial starters; i.e. a national market available baker's yeast and a bread-making starter produced in China and sold in Asia and Europe.

The tests have been carried out using experimental dough with the following composition: 500 g hard wheat semola, 250 ml water, 15 g sea salt, 3 g sugar added to 7.5 ml yeast paste at concentration of 1,0x10¹⁰ cell/ml.

Different dough have been placed in parafilm covered 1000 ml graded cylinders and incubated at 35°C for 4 hours. Every hour the yeasting power was measured.

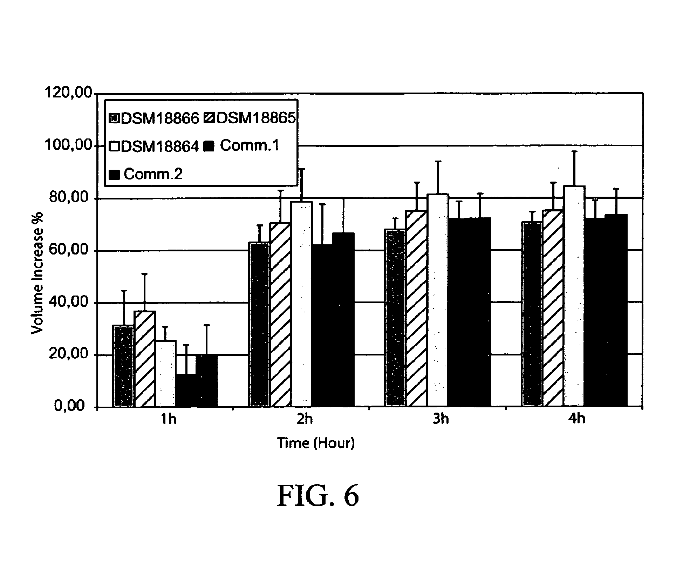

The results expressed as % volumetric increase are reported in tables 4, 5, 6 and 7 and Figure 6. Variance analysis (ANOVA) was used in order to evaluate the significance of different yeasting power at each hour. The comparison of six replicates averages has been carried out using Duncan test (P ≤ 0,05).

**Table 4: % Volumetric increase induced by yeast strains after 1 hour of incubation**

| | DSM 18866 | DSM 18865 | DSM 18864 | CS 117 | ZP1 |
|---|---|---|---|---|---|
| | 21.81 | 37.78 | 26.09 | 6.52 | 2.13 |
| | 34.04 | 15.22 | 21.74 | 36.17 | 13.73 |
| | 57.39 | 52.36 | 24.44 | 13.04 | 19.57 |
| | 36.75 | 51.06 | 36.52 | 25.12 | 20.44 |
| | 28.82 | 35.87 | 29.31 | 11.74 | 23.38 |
| | 21.74 | 26.12 | 21.74 | 7.93 | 36.96 |
| median | 31.43 | 36.82 | 25.27 | 12.39 | 20.00 |
| *S.* d. | 13.26 | 14.33 | 5.62 | 11.56 | 11.45 |

**Table 5: % Volumetric increase induced by yeast strains after 2 hours of incubation**

| | DSM 18866 | DSM 18865 | DSM 18864 | CS 117 | ZP1 |
|---|---|---|---|---|---|
| | 60.94 | 77.78 | 68.85 | 28.26 | 36.17 |
| | 57.43 | 58.70 | 56.52 | 70.21 | 65.24 |
| | 69.57 | 63.09 | 86.67 | 64.78 | 73.91 |
| | 75.21 | 82.98 | 84.78 | 59.80 | 60.87 |
| | 64.42 | 88.41 | 72.42 | 64.35 | 67.96 |
| | 61.72 | 62.16 | 87.83 | 47.58 | 70.44 |
| median | 63.07 | 70.43 | 78.60 | 62.07 | 66.60 |
| s.d. | 6.48 | 12.46 | 12.44 | 15.51 | 13.61 |

**Table 6: % Volumetric increase induced by yeast strains after 3 hours of incubation**

| | DSM 18866 | DSM 18865 | DSM 18864 | CS 117 | ZP1 |
|---|---|---|---|---|---|
| | 67.48 | 82.22 | 73.43 | 73.91 | 55.32 |
| | 68.09 | 65.22 | 60.87 | 74.47 | 71.67 |
| | 73.91 | 66.09 | 93.33 | 71.74 | 80.44 |
| | 75.21 | 87.23 | 86.09 | 72.18 | 66.96 |
| | 68.00 | 88.41 | 76.72 | 69.57 | 72.73 |
| | 64.35 | 68.02 | 92.61 | 56.39 | 80.87 |
| median | 68.04 | 75.12 | 81.41 | 71.96 | 72.20 |
| s. d. | 4.17 | 10.92 | 12.59 | 6.75 | 9.50 |

**Table 7: % Volumetric increase induced by yeast strains after 4 hours of incubation**

| | DSM 18866 | DSM 18865 | DSM 18864 | CS 117 | ZP1 |
|---|---|---|---|---|---|
| | 67.48 | 82.22 | 73.84 | 78.26 | 55.32 |
| | 70.21 | 65.22 | 60.87 | 74.47 | 71.67 |
| | 73.91 | 67.38 | 95.56 | 73.91 | 82.61 |
| | 75.21 | 87.23 | 89.13 | 70.13 | 67.39 |
| | 71.11 | 88.41 | 79.74 | 69.57 | 75.32 |
| | 64.35 | 68.02 | 93.91 | 57.71 | 80.87 |
| median | 70.66 | 75.12 | 84.44 | 72.02 | 73.50 |
| dev.st | 4.03 | 10.70 | 13.38 | 7.10 | 10.02 |

The evaluations shown that the isolated yeast strains display interesting industrial characteristics. Particularly, during the first yeasting hour the yeasting power differences between DSM 18865 strain (TS60) and two commercial starters are particularly significant. In fact the dough obtained using DSM 18865 (TS60) strain has 20 % higher volume than those obtained using commercial yeasts (Figure 7). Moreover during the second and until the fourth hour DSM 18864 (LS 250) named strain has shown a yeasting power higher than others with meaningful differences at the second hour.

### BIBLIOGRAPHY

- Casiraghi E., Rollini M., Zardi M., Manzoni M. (2006). Tecnica Molitoria, (10): 1068-1074;
- Catzeddu P., Sanna M., Farris G.A. (2002). Tecnica Molitoria (08): 788-798;
- Restuccia C., Cascone C., Randazzo C., Caggia C. (2005). Industrie Alimentari, XLIV (5): 483-487;
- Ricciardi A., Paraggio M., Salzano G., Andreotti G. (2002). Tecnica Molitoria (08): 780-787;
- Severini C., De Pilli T., Cannito G., Baiano A., Peri G. (2002). Tecnica Molitoria (06): 560-574;
- Solieri L., De Vero L., Pulvirenti A. (2003). Industrie Alimentari, XLII (10): 971-978;
- Tannini E., Paoloni M., Papa R., Clementi F. (2006). Tecnica Molitoria (06): 650-658.

## Claims

1. Microbial starter for bakery products based on Saccharomyces cerevisiae yeast strains comprising or consisting of at least one of yeast strains selected from the group consisting of Saccharomyces cerevisiae DSM 18864, Saccharomyces cerevisiae DSM 18865 and Saccharomyces cerevisiae DSM 18866 or a combination thereof.

2. Microbial starter according to claim 1, comprising or consisting of a combination of Saccharomyces cerevisiae DSM 18864 and Saccharomyces cerevisiae DSM 18865 or Saccharomyces cerevisiae DSM 18865 and Saccharomyces cerevisiae DSM 18866.

3. Microbial starter according to anyone of the claims 1-2 wherein the strains are in a 1:1 ratio at a cell density of 1x10¹⁰ cfu/g at fermentation starting point.

4. Microbial starter according to anyone of the claims 1-3 wherein the strains are in a liquid, freeze-dried, frozen, dried form.

5. Use of the microbial starter according to anyone of the preceding claims for wheat flour yeasting and production of bakery products, preferably bread.

6. Use according to claim 5 wherein the wheat flours are selected from the group consisting of hard wheat semola flour, soft wheat flour 0 and 00 or mixtures thereof.

7. Use according to anyone of the claims 5-6 wherein the use dose is equal to 1 % (w/w).

8. Process for the preparation of the microbial starter for bakery products as defined in claims 1-4 comprising the following steps of:
a) culture propagating of at least one of the yeast strains selected from the group consisting of Saccharomyces cerevisiae DSM 18864, Saccharomyces cerevisiae DSM 18865 and Saccharomyces cerevisiae DSM 18866 or a combination thereof;
b) wheat flour kneading containing the culture of bacterial strains of a) step at cellular density of 1x10¹⁰ cfu/g at 1:1 strain ratio;
c) allowing the fermentation to be performed for 1.15-1.30 hours at 35°-40°C.

9. Process according to claim 8 wherein the wheat flour is 100 % semola wheat flour or 100 % 00 wheat flour or 80 % semola wheat flour and 20 % 0 or 00 wheat flour or 50 % semola wheat flour and 50 % 0 or 00 wheat flour.

10. Process according to anyone of the claims 8-9 wherein % weight of water in b) step dough is 50 % - 60 %.

11. Process according to anyone of the claims 8-10 further comprising a d) step for drying, freeze-drying, drying or emulsifying in liquid phase of the starter obtained in a) step.

12. Microbial starter composition obtainable by using the process as defined according to anyone of the claims 8-11.

13. Process for the preparation of bakery products comprising the following steps of:
a) kneading the wheat flour with water containing microbial starter inoculum obtainable using the process as defined according to each of claims 8-11;
b) allowing the fermentation to be carried out for approximately 1.15-1.30 hours at 35°-40°C;
c) cooking for 40-50 minutes at 220-240°C.

14. Process according to claim 13, wherein the wheat flour is 100 % semola wheat flour or 100 % 00 wheat flour or 80 % semola wheat flour and 20 % 0 or 00 wheat flour or 50 % semola wheat flour and 50 % 0 or 00 wheat flour.

15. Process according to anyone of the claims 13-14 wherein % weight of water in the dough of a) step is 50 % - 60 %.

16. Process according to anyone of the claims 13-15, wherein the bakery product is bread.

## Patentansprüche

1. Mikrobieller Starter für Backprodukte auf der Basis von Saccharomyces cerevisiae-Hefestämmen, der mindestens einen von Hefestämmen, die aus der Gruppe, welche aus Saccharomyces cerevisiae DSM 18864, Saccharomyces cerevisiae DSM 18865 und Saccharomyces cerevisiae DSM 18866 oder einer Kombination davon besteht, ausgewählt sind, umfasst oder daraus besteht.

2. Mikrobieller Starter nach Anspruch 1, der eine Kombination von Saccharomyces cerevisiae DSM 18864 und Saccharomyces cerevisiae DSM 18865 oder Saccharomyces cerevisiae DSM 18865 und Saccharomyces cerevisiae DSM 18866 umfasst oder daraus besteht.

3. Mikrobieller Starter nach irgendeinem der Ansprüche 1-2, wobei die Stämme in einem Verhältnis von 1:1 bei einer Zelldichte von 1 x 10¹⁰ cfu/g zu Fermentationsbeginn vorliegen.

4. Mikrobieller Starter nach irgendeinem der Ansprüche 1-3, wobei die Stämme in flüssiger, gefriergetrockneter, gefrorener oder getrockneter Form vorliegen.

5. Verwendung des mikrobiellen Starters nach irgendeinem der vorhergehenden Ansprüche für die Hefebehandlung von Weizenmehlen und die Herstellung von Backprodukten, vorzugsweise Brot.

6. Verwendung nach Anspruch 5, wobei die Weizenmehle aus der Gruppe, welche aus Hartweizen-Semolamehl, Weichweizenmehl Nr. 0 und 00 oder Mischungen davon besteht, ausgewählt sind.

7. Verwendung nach irgendeinem der Ansprüche 5-6, wobei die Verwendungsdosis 1% (Gew./Gew.) beträgt.

8. Verfahren zur Herstellung des mikrobiellen Starters für Backprodukte wie in den Ansprüchen 1-4 definiert, umfassend die folgenden Schritte:
a) Vermehren in Kultur von mindestens einem der Hefestämme, die aus der Gruppe, welche aus Saccharomyces cerevisiae DSM 18864, Saccharomyces cerevisiae DSM 18865 und Saccharomyces cerevisiae DSM 18866 oder einer Kombination davon besteht, ausgewählt sind;
b) Kneten von Weizenmehl, welches die Kultur der Bakterienstämme von Schritt a) bei einer Zelldichte von 1 x 10¹⁰ cfu/g in einem 1:1-Stammverhältnis enthält;
c) Gestatten der Durchführung einer Fermentation für 1,15-1,30 Stunden bei 35°-40°C.

9. Verfahren nach Anspruch 8, wobei das Weizenmehl 100 % Semola-Weizenmehl oder 100 % 00-Weizenmehl oder 80 % Semola-Weizenmehl und 20 % 0- oder 00-Weizenmehl oder 50 % Semola-Weizenmehl und 50 % 0- oder 00-Weizenmehl ist.

10. Verfahren nach irgendeinem der Ansprüche 8-9, wobei die Gewichtsprozent Wasser im Teig von Schritt b) 50 % bis 60 % betragen.

11. Verfahren nach irgendeinem der Ansprüche 8-10, welches ferner einen Schritt d) zum Trocknen, Gefriertrocknen, Trocknen oder Emulgieren in flüssiger Phase des in Schritt a) erhaltenen Starters umfasst.

12. Mikrobielle Starter-Zusammensetzung, erhältlich durch Anwendung des Verfahrens wie in irgendeinem der Ansprüche 8-11 definiert.

13. Verfahren zur Herstellung von Backprodukten, umfassend die folgenden Schritte:
a) Kneten des Weizenmehls mit Wasser, enthaltend ein Impfgut des mikrobiellen Starters, der durch Anwendung des Verfahrens wie in irgendeinem der Ansprüche 8-11 definiert erhältlich ist;
b) Gestatten der Durchführung einer Fermentation für etwa 1,15-1,30 Stunden bei 35°-40°C;
c) Erwärmen für 40-50 Minuten bei 220°-240°C.

14. Verfahren nach Anspruch 13, wobei das Weizenmehl 100 % Semola-Weizenmehl oder 100 % 00-Weizenmehl oder 80 % Semola-Weizenmehl und 20 % 0- oder 00-Weizenmehl oder 50 % Semola-Weizenmehl und 50 % 0- oder 00-Weizenmehl ist.

15. Verfahren nach irgendeinem der Ansprüche 13-14, wobei die Gewichtsprozent Wasser in dem Teig von Schritt a) 50 % bis 60 % betragen.

16. Verfahren nach irgendeinem der Ansprüche 13-15, wobei das Backprodukt Brot ist.

## Revendications

1. Levain (« starter ») microbien pour produits de boulangerie à base de souches de levure Saccharomyces cerevisiae, comprenant ou se composant d'au moins une des souches de levure sélectionnées dans le groupe composé de Saccharomyces cerevisiae DSM 18864, de Saccharomyces cerevisiae DSM 18865 et de Saccharomyces cerevisiae DSM 18866, ou d'une combinaison de celles-ci.

2. Levain microbien selon la revendication 1, comprenant ou se composant d'une combinaison de Saccharomyces cerevisiae DSM 18864 et de Saccharomyces cerevisiae DSM 18865, ou de Saccharomyces cerevisiae DSM 18865 et de Saccharomyces cerevisiae DSM 18866.

3. Levain microbien selon la revendication 1 ou 2, dans lequel les souches sont présentes selon un rapport de 1:1, avec une densité cellulaire de 1 x 10¹⁰ ufc/g au point de départ de la fermentation.

4. Levain microbien selon l'une quelconque des revendications 1 à 3, dans lequel les souches se présentent sous une forme liquide, lyophilisée, congelée ou séchée.

5. Utilisation du levain microbien selon l'une quelconque des revendications précédentes pour l'ensemencement de farine de blé et la production de produits de boulangerie, de préférence de pain.

6. Utilisation selon la revendication 5, dans laquelle les farines de blé sont sélectionnées dans le groupe composé de la farine de semoule de blé dur, de la farine de blé tendre 0 et 00, ou de leurs mélanges.

7. Utilisation selon la revendication 5 ou 6, dans laquelle la dose d'utilisation est égale à 1 % (en poids).

8. Procédé de préparation du levain microbien pour produits de boulangerie tel que défini dans les revendications 1 à 4, comprenant les étapes suivantes consistant à :
a) propager par culture au moins une des souches de levure sélectionnées dans le groupe composé de Saccharomyces cerevisiae DSM 18864, de Saccharomyces cerevisiae DSM 18865 et de Saccharomyces cerevisiae DSM 18866, ou d'une combinaison de celles-ci ;
b) pétrir la farine de blé contenant la culture de souches bactériennes de l'étape a) selon une densité cellulaire de 1x10¹⁰ufc/g, avec un rapport des souches de 1:1 ;
c) laisser la fermentation s'opérer pendant 1h15 à 1h30 heures entre 35 et 40 °C.

9. Procédé selon la revendication 8, dans lequel la farine de blé est à 100 % de la farine de semoule de blé ou à 100 % de la farine de blé 00, ou à 80 % de la farine de semoule de blé et à 20 % de la farine de blé 0 ou 00, ou à 50 % de la farine de semoule de blé et à 50 % de la farine de blé 0 ou 00.

10. Procédé selon la revendication 8 ou 9, dans lequel le % en poids d'eau dans la pâte de l'étape b) est compris entre 50 % et 60 %.

11. Procédé selon l'une quelconque des revendications 8 à 10, comprenant en outre une étape d) pour sécher, lyophiliser, sécher ou émulsionner en phase liquide le levain obtenu à l'étape a).

12. Composition de levain microbien pouvant être obtenue par le procédé tel que défini dans l'une quelconque des revendications 8 à 11.

13. Procédé de préparation de produits de boulangerie, comprenant les étapes suivantes consistant à :
a) pétrir la farine de blé avec de l'eau contenant l'inoculum de levain microbien pouvant être obtenu en utilisant le procédé tel que défini selon chacune des revendications 8 à 11;
b) laisser la fermentation s'opérer pendant environ 1h15 à 1h30 entre 35 et 40°C;
c) cuire pendant 40 à 50 minutes entre 220 et 240°C.

14. Procédé selon la revendication 13, dans lequel la farine de blé est à 100 % de la farine de semoule de blé ou à 100 % de la farine de blé 00, ou à 80 % de la farine de semoule de blé et à 20 % de la farine de blé 0 ou 00, ou à 50 % de la farine de semoule de blé et à 50 % de la farine de blé 0 ou 00.

15. Procédé selon la revendication 13 ou 14, dans lequel le pourcentage en poids d'eau dans la pâte de l'étape a) est compris entre 50 %et60%.

16. Procédé selon l'une quelconque des revendications 13 à 15, dans lequel le produit de boulangerie est du pain.
